(19) Europäisches Patentamt
European Patent Office
Office européen des brevets

(11) Veröffentlichungsnummer: **0 263 059 B1**

# (12) EUROPÄISCHE PATENTSCHRIFT

(45) Veröffentlichungstag der Patentschrift: **11.11.92**

(51) Int. Cl.$^5$: **C07C 237/00**, C07D 265/33, A61K 49/00

(21) Anmeldenummer: **87730111.9**

(22) Anmeldetag: **23.09.87**

Die Akte enthält technische Angaben, die nach dem Eingang der Anmeldung eingereicht wurden und die nicht in dieser Patentschrift enthalten sind.

## (54) Amid-Komplexe.

(30) Priorität: **26.09.86 DE 3633245**
**26.09.86 DE 3633246**

(43) Veröffentlichungstag der Anmeldung:
**06.04.88 Patentblatt 88/14**

(45) Bekanntmachung des Hinweises auf die Patenterteilung:
**11.11.92 Patentblatt 92/46**

(84) Benannte Vertragsstaaten:
**AT BE CH DE ES FR GB GR IT LI LU NL SE**

(56) Entgegenhaltungen:
**EP-A- 0 130 934**
**EP-A- 0 169 299**
**WO-A-86/02841**
**DE-A- 3 401 052**
**FR-A- 2 045 987**
**US-A- 4 859 451**

**ZEITSCHRIFT FUER NATURFORSCHUNG, Band 40B, Nr. 11, 4. März 1985, Seiten 1558-1562; G. SOSNOVSKY et al, "Spin labeled chelating agents and their gadolinium complexes as contrast enhancing agents for NMR imaging"**

**Radiocontrast Agents, Editor M. Sovak, D. Noninionic Contrast Media (G. B. Hoey and K. R. Smith), pages 54-56**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Gries, Heinz, Dr.**
**Helmstedter Strasse 19**
**W-1000 Berlin 31(DE)**
Erfinder: **Radüchel, Bernd, Dr.**
**Gollanczstrasse 132**
**W-1000 Berlin 28(DE)**
Erfinder: **Weinmann, Hanns-Joachim, Dr.**
**Ahornstrasse 31**
**W-1000 Berlin 41(DE)**
Erfinder: **Mützel, Wolfgang, Dr.**
**Weddigenweg 74**
**W-1000 Berlin 45(DE)**
Erfinder: **Speck, Ulrich, Prof. Dr.**
**Benediktiner Strasse 50**
**W-1000 Berlin 28(DE)**

**EP 0 263 059 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft den in den Patentansprüchen gekennzeichneten Gegenstand, d.h. neue Komplexbildner, Komplexe und Komplexsalze, diese Verbindungen enthaltende Mittel, ihre Verwendung in der Diagnostik, Verfahren zur Herstellung dieser Verbindungen und Mittel, sowie ein hierzu geeignetes Ausgangsmaterial.

Metallkomplexe sind schon zu Beginn der 50er Jahre als Kontrastmittel für die Radiologie in Betracht gezogen worden. Die damals eingesetzten Verbindungen waren aber derart toxisch, dass eine Verwendung beim Menschen nicht in Frage kam. Es war daher sehr überraschend, dass sich bestimmte Komplexsalze als ausreichend verträglich erwiesen haben, sodass eine routinemässige Anwendung am Menschen für diagnostische Zwecke in Erwägung gezogen werden konnte.

In den Patentanmeldungen EP 71564, EP 130934, DE-OS 3401052, PCT WO 86/02841 und PCT WO 86/02005 sind neuerdings Komplexe und Komplexsalze als Diagnostika, vorwiegend als NMR-Diagnostika, vorgestellt worden.

Alle bisher bekannten Komplexe und deren Salze bereiten bei ihrer klinischen Anwendung Probleme im Hinblick auf die Verträglichkeit und/oder Selektivität der Bindung und/oder Stabilität. Ausserdem weisen die in den beiden zuletzt genannten Patentanmeldungen vorgestellten Komplexe eine zu hohe Lipophilie auf. Diese Probleme sind umso ausgeprägter, je höher die aus den Komplexbildnern abgeleiteten Produkte dosiert werden müssen. Die an und für sich nützliche Anwendung schwerer Elemente als Bestandteile von parenteral zu verabreichenden Röntgenkontrastmitteln scheiterte bisher an der ungenügenden Verträglichkeit derartiger Verbindungen. Bei den bisher für die Kernspintomographie vorgeschlagenen oder geprüften paramagnetischen Substanzen ist der Abstand zwischen der wirksamen und der im Tierexperiment toxischen Dosis relativ eng und/oder sie weisen eine geringe Organspezifität und/oder Stabilität und/oder kontrastverstärkende Wirkung auf und/oder ihre Verträglichkeit ist unzureichend.

Es besteht daher für vielfältige Zwecke ein Bedarf an vor allem besser verträglichen, aber auch stabilen, gut löslichen und hinreichend selektiven Komplexverbindungen.

Der Erfindung liegt somit die Aufgabe zugrunde, diese Verbindungen und Mittel zur Verfügung zu stellen, sowie ein Verfahren zu ihrer Herstellung sowie ein geeignetes Ausgangsmaterial zu schaffen. Diese Aufgabe wird durch die Erfindung gelöst.

Es wurde gefunden, daß sich Verbindungen, die aus dem Anion eines komplexbildenden Amids und einem oder mehreren Zentralionen eines Elements der Ordnungszahlen 21-29, 31, 32, 38, 39, 42-44, 49, 57-83 und gegebenenfalls einem oder mehreren Kationen einer anorganischen und/oder organischen Base oder Aminosäure bestehen, überraschenderweise hervorragend zur Herstellung vom NMR-, Röntgen- und Radio-Diagnostika eignen.

Die erfindungsmäßigen Verbindungen werden durch die allgemeine Formel I beschrieben:
Physiologisch verträgliche Verbindungen der allgemeinen Formel I

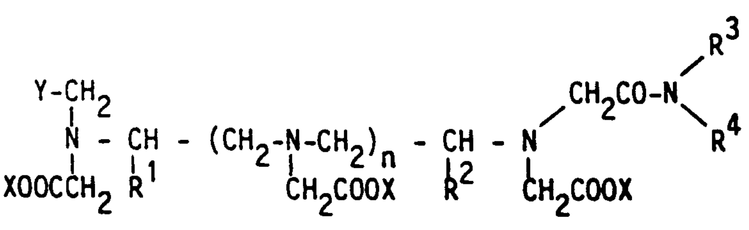

(I),

worin

n die Ziffern 0, 1 oder 2,

$R^1$ und $R^2$ Wasserstoffatome, niedere Alkylgruppen, Phenylgruppen, Benzylgruppen oder, wenn n für die Ziffer 0 steht, auch gemeinsam eine Trimethylen- oder eine Tetramethylengruppe,

$R^3$ ein gesättigter, ungesättigter, gerad- oder verzweigtkettiger oder cyclischer aliphatischer Kohlenwasserstoffrest mit bis zu 16 C-Atomen, oder, wenn $R^4$ ein Wasserstoffatom ist, eine Cycloalkyl- oder eine gegebenenfalls durch eine oder mehrere Di-$C_1$-$C_6$-alkylamino- oder durch eine oder mehrere $C_1$-$C_6$-Alkoxygruppen substituierte Aryl- oder Aralkylgruppe,

$R^4$ ein Wasserstoffatom, ein gesättigter, ungesättigter, gerad- oder verzweigtkettiger oder cyclischer Kohlenwasserstoffrest mit bis zu 16 C-Atomen, mit der Maßgabe, daß - wenn $R^1$ und $R^2$ für Wasserstoffatome stehen - $R^3$ und $R^4$ nicht gleichzeitig für gesättigte, ungesättigte, gerad- oder verzweigtkettige aliphatische Kohlenwasserstoffreste mit bis zu 16 C-Atomen stehen, oder

$R^3$ und $R^4$ gemeinsam eine gesättigten oder ungesättigten, gegebenenfalls durch einen oder mehrere $C_1$-$C_6$-Alkyl-, $C_1$-$C_5$-Hydroxyalkyl-, einen gegebenenfalls hydroxylierten oder $C_1$-$C_6$-alkoxylierten $C_2$-$C_6$-Acyl-, Hydroxy-, Carbamoyl-, Carbamoyl-substituierten $C_1$-$C_6$-Alkyl-, am Carbamoyl-Stickstoff durch einen oder zwei $C_1$-$C_6$-Alkylrest(e) - die auch einen gegebenenfalls ein Sauerstoffatom enthaltenen Ring bilden können - substituierten Carbamoyl-, oder einen $C_1$-$C_6$-Acylamino- oder $C_1$-$C_6$-Alkylaminorest substituierten 5- oder 6-Ring, der gegebenenfalls ein weiteres Stickstoff-, Sauerstoff-, Schwefelatom oder eine Carbonylgruppe enthält,

X ein Wasserstoffatom und/oder ein Metallionenäquivalent,

Y eine COOX- oder

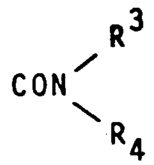

- Gruppe

bedeuten, sowie deren Salze mit organischen und/oder anorganischen Basen.

Verbindungen der allgemeinen Formel I mit X in der Bedeutung von Wasserstoff werden als Komplexbildner und mit mindestens zwei der Substituenten X in der Bedeutung eines Metallionenäquivalents als Metallkomplexe bezeichnet.

Das Element der oben genannten Ordnungszahl, welches das Zentralion des physiologisch verträglichen Komplexsalzes bildet, kann für den angestrebten Verwendungszweck des erfindungsgemäßen diagnostischen Mittels selbstverständlich auch radioaktiv sein.

Ist das erfindungsgemäße Mittel zur Anwendung in der NMR-Diagnostik bestimmt (siehe die Europäische Patentanmeldung Nr. 71564), so muß das Zentralion des Komplexsalzes paramagnetisch sein. Dies sind insbesondere die zwei- und dreiwertigen Ionen der Elemente der Ordnungszahlen 21-29, 42, 44 und 57-70. Geeignete Ionen sind beispielsweise das Chrom(III)-, Mangan(II)-, Eisen(II)-, Cobalt(II)-, Nickel(II)-, Kupfer(II)-, Praseodym(III), Neodym(III)-, Samarium(III)- und Ytterbium(III)-ion. Wegen ihres sehr starken magnetischen Moments sind besonders bevorzugt das Gadolinium(III)-, Terbium(III)-, Dysprosium(III)-, Holmium(III)-, Erbium(III)- und Eisen(III)-ion.

Für die Verwendung der erfindungsgemäßen Mittel in der nuklearmedizinischen Diagnostik muß das Zentralion radioaktiv sein. Geeignet sind zum Beispiel Radioisotope der Elemente Kupfer, Kobalt, Gallium, Germanium, Yttrium, Strontium, Technetium, Indium, Ytterbium, Gadolinium, Samarium und Iridium.

Ist das erfindungsgemäße Mittel zur Anwendung in der Röntgen-Diagnostik und -Therapie bestimmt, so muß sich das Zentralion von einem Element höherer Ordnungszahl ableiten, um eine ausreichende Absorption der Röntgenstrahlen zu erzeilen. Es wurde gefunden, daß zu diesem Zweck diagnostische Mittel, die ein physiologisch verträgliches Komplexsalz mit Zentralionen von Elementen der Ordnungszahlen zwischen 21-29, 42, 44, 57-83 enthalten, geeignet sind; dies sind beispielsweise das Lanthan(III)-ion und die oben genannten Ionen der Lanthanidenreihe.

Als Alkylsubstituenten $R^1$ und $R^2$ kommen Kohlenwasserstoffe mit 1 - 8, vorzugsweise 1 - 4 C-Atomen, wie z.B. Methyl-, Ethyl-, Propyl-, Isopropyl, n-, sek.- oder tert.-Butyl-, Isobutyl-, Pentyl- und Hexyl-Reste, in Betracht.

Als Alkylsubstituenten $R^3$ und $R^4$ kommen gesättigte, ungesättigte, gerad- oder verzweigtkettige oder cyclische Kohlenwasserstoffe mit bis zu 16 C-Atomen, vorzugsweise gesättigte Kohlenwasserstoffe mit 1 bis 10 C-Atomen, insbesondere gesättigte Kohlenwasserstoffe mit 1 bis 5 C-Atomen in Betracht. Beispielsweise seien genannt: Methyl-, Ethyl-, Propyl-, Isopropyl-, Butyl-, Isobutyl-, Pentyl-, Cyclopentyl-, Cyclohexyl-, Propenyl-.

Falls $R^4$ für ein Wasserstoffatom steht, kann $R^3$ auch eine gegebenenfalls durch eine oder mehrere (bis zu drei) Di-$C_1$-bis $C_6$-alkylamino- oder durch eine oder mehrere (bis zu drei) $C_1$- bis $C_6$-Alkoxygruppen substituierte $C_6$-$C_{10}$-Aryl- oder $C_1$-$C_{10}$-Ar-$C_1$-$C_6$-alkylgruppe, beispielsweise Phenyl- oder Benzylgruppe bedeuten.

Der durch $R^3$ und $R^4$ unter Einschluss des Amid-Stickstoffs gebildete heterocyclische 5- oder 6-Ring kann gesättigt, ungesättigt und/oder substituiert sein und gegebenenfalls ein Stickstoff-, Sauerstoff-, Schwefelatom oder eine Carbonylgruppe enthalten.

Der Heterocyclus kann substituiert sein durch eine Hydroxy-, eine $C_1$-$C_6$-Alkylgruppe, beispielsweise Methyl, Ethyl, Propyl, Isopropyl, Butyl, eine $C_1$-$C_5$-Hydroxyalkylgruppe, beispielsweise Hydroxymethyl,

Hydroxyethyl, oder durch eine $C_2$-$C_6$-Acylgruppe, zum Beispiel Acetyl, Propionyl, die gegebenenfalls durch eine Hydroxy- oder $C_1$-$C_6$-Alkoxygruppe, beispielsweise Methoxy, Ethoxy, substituiert sein kann.

Als weiterer Substituent sei die Carbamoylgruppe genannt, die direkt oder durch eine $C_1$-$C_6$-Alkylengruppe, beispielsweise Methylen, Ethylen, Propylen getrennt am Heterocyclus gebunden ist und gegebenenfalls durch einen oder zwei $C_1$-$C_6$-Alkylrest(e), beispielsweise Methyl, Ethyl, Propyl, Isopropyl, die gegebenenfalls einen Ring wie zum Beispiel einen Pyrrolidin- oder Piperidin-Ring bilden, am Stickstoff substituiert ist. Der Carbamoyl-Stickstoff kann auch Bestandteil eines Morpholin-Ringes sein.

Als weiterer möglicher Substituent am Heterocyclus sei eine gegebenenfalls $C_1$-$C_6$-alkylierte oder $C_1$-$C_6$ acylierte primäre oder sekundäre Aminogruppe, wie beispielsweise die Methyl-, Ethyl-, Acetyl-, Propionyl-Aminogruppe, genannt.

Wenn der Heterocyclus substituiert ist, beträgt die Gesamtzahl der Substituenten 1 bis 3.

Als geeignete Heterocyclen seien beispielhaft genannt:

der Pyrrolidinyl-, Piperidyl-, Pyrazolidinyl-, Pyrrolinyl-, Pyrazolinyl-, Piperazinyl-, Morpholinyl-, Imidazolidinyl-, Oxazolidinyl-, Thiazolidinyl-Ring.

Wenn nicht alle aziden Wasserstoffatome durch das Zentralion substituiert werden, können ein, mehrere oder alle verbleibenden Wasserstoffatom(e) durch Kationen anorganischer und/oder organischer Basen oder Aminosäuren ersetzt sein. Geeignete anorganische Kationen sind beispielsweise das Lithiumion, das Kaliumion, das Calciumion und insbesondere das Natriumion. Geeignete Kationen organischer Basen sind unter anderem solche von primären, sekundären oder tertiären Aminen, wie zum Beispiel Ethanolamin, Diethanolamin, Morpholin, Glucamin, N,N-Dimethylglucamin und insbesondere N-Methylglucamin. Geeignete Kationen von Aminosäuren sind beispielsweise die des Lysins, des Arginins und des Ornithins.

Die Einführung von Amidgruppen zur Herstellung der erfindungsgemässen Komplexbildner, d.h. von Verbindungen der allgemeinen Formel I mit X in der Bedeutung von Wasserstoff, erfolgt durch partielle Umwandlung von aktivierten Carboxylgruppen in Amidgruppen der -entsprechend dem gewünschten Endprodukt- jeweils geeigneten Tetra-, Penta- bzw. Hexacarbonsäuren. Für diesen Prozess kommen alle dem Fachmann bekannten Synthesemöglichkeiten in betracht.

Ein Beispiel hierfür ist die Umsetzung der Anhydride oder Ester der allgemeinen Formeln II, IV, V and VI

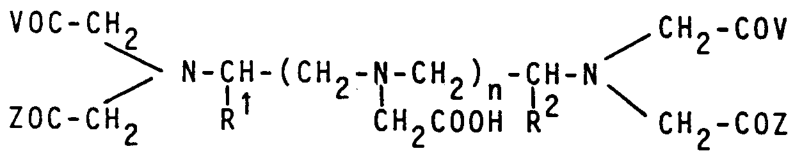

(II)

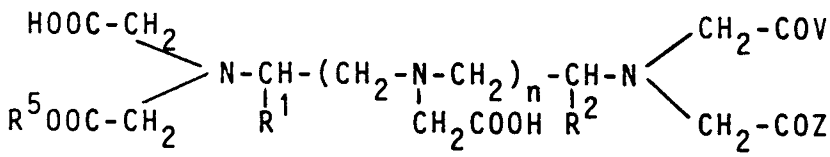

(IV)

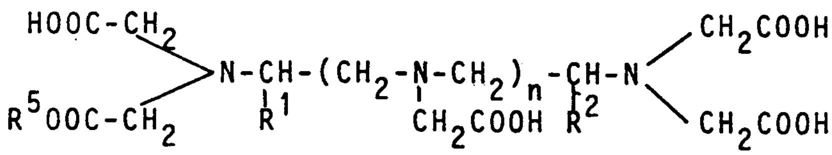

(V)

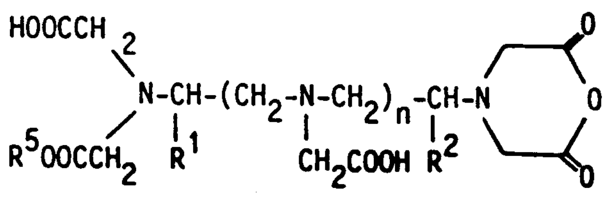

(VI),

Worin

$R^1$, $R^2$ und n die oben angegebenen Bedeutungen haben, V and Z gemeinsam ein Sauerstoffatom oder V eine Hydroxygruppe und Z die Gruppierung $OR^5$ und $R^5$ einen $C_1$-$C_6$-Alkylrest darstellen, mit Aminen der allgemeinen Formel III

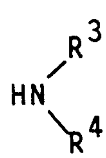

(III),

worin

$R^3$ und $R^4$ die oben genannten Bedeutungen haben.

Als geeignete Amine seien beispielsweise genannt:

Dimethylamin, Diethylamin, Di-n-propylamin, Diisopropylamin, Di-n-butylamin, Diisobutylamin, Di-sek.butylamin, N-Methyl-n-propylamin, Dioctylamin, Dicyclohexylamin, N-Ethylcyclohexylamin, Diisopropenylamin, Benzylamin, Anilin, 4-Methoxyanilin, 4-Dimethylaminoanilin, 3,5-Dimethoxyanilin, Morpholin, Pyrrolidin, Piperidin, N-Methyl-piperazin, N-Ethylpiperazin, N-(2-Hydroxyethyl)-piperazin, N-(Hydroxymethyl)-piperazin, Piperazinoessigsäureisopropylamid, N-(Piperazinomethylcarbonyl)-morpholin, N-(Piperazinomethylcarbonyl)-pyrrolidin, 2-(2-Hydroxymethyl)-piperidin, 4-(2-Hydroxyethyl)-piperidin, 2-Hydroxymethylpiperidin, 4-Hydroxymethylpiperidin, 2-Hydroxymethyl-pyrrolidin, 3-Hydroxy-piperidin, 4-Hydroxy-piperidin, 3-Hydroxy-pyrrolidin, 4-Piperidon, 3-Pyrrolin, Piperidin-3-carbonsäureamid, Piperidin-4-carbonsäureamid, Piperidin-3-carbonsäurediethylamid, Piperidin-4-carbonsäuredimethylamid, 2,6-Dimethylpiperidin, 2,6-Dimethylmorpholin, N-Acetyl-piperazin, N-(2-Hydroxy-propionyl)-piperazin, N-(3-Hydroxy-propionyl)-piperazin, N-(Methoxyacetyl)-piperazin, 4-(N-Acetyl,N-methylamino)-piperidin, Piperidin-4-carbonsäure-(3-oxapentamethylen)-amid, Piperidin-3-carbonsäure-(3-oxapentamethylen)-amid, N-(N′,N′-Dimethyl-carbamoyl)-piperazin, Pyrazolin, Pyrazolidin, Imidazolin, Oxazolidin, Thiazolidin.

Die Verseifung gegebenenfalls noch vorhandener Estergruppen erfolgt nach den dem Fachmann bekannten Verfahren, beispielsweise durch alkalische Hydrolyse.

Die Herstellung der Säureanhydride der allgemeinen Formel II kann nach bekannten Verfahren erfolgen, z.B. nach der im US-Patent 3.660.388 bzw. in DE-OS 16.95.050 beschriebenen Verfahrensweise mit Acetanhydrid in Pyridin. In bestimmten Fällen ist es jedoch von besonderem Vorteil, die Wasserabspaltung mit Carbodiimiden in einem geeigneten Lösungsmittel, wie z.B. Dimethylformamid oder Dimethylacetamid, schonend vorzunehmen.

Die Herstellung der erfindungsgemäßen Monoanhydride der allgemeinen Formel VI soll am Beispiel des Monoanhydrids des Diethylentriaminpentaessigsäure-ethylesters, ausgehend vom Monoethylester der DTPA (J. Pharm. Sci. 68, 1979, 194), beschrieben werden:

$N^3$-(2,6-Dioxomorpholinoethyl)-$N^6$-(ethoxycarbonylmethyl)-3,6-diazaoctandisäure

Eine Suspension von 21.1 g (50 mMol) $N^3$,$N^6$-Bis-(carboxymethyl)-$N^9$-(ethoxycarbonylmethyl)-3,6,9-triazaundecandisäure in 250 ml Essigsäureanhydrid läßt man nach Zugabe von 42.2 ml Pyridin drei Tage bei Raumtemperatur rühren. Dann saugt man den Niederschlag ab, wäscht ihn dreimal mit je 50 ml Essigsäureanhydrid und verrührt ihn anschließend mehrere Stunden mit absolutem Diethylether. Nach Absaugen, Waschen mit absolutem Diethylether und Trocknen im Vakuum bei 40 °C erhält man 18.0 g (= 89 % der Theorie) eines weißen Pulvers vom Schmelzpunkt 195-196 °C.

| Analyse (bezogen auf wasserfreie Substanz): | | | |
|---|---|---|---|
| C 47.64 | H 6.25 | N 10.42 | (Ber.) |
| C 47.54 | H 6.30 | N 10.22 | (Gef.) |

Die Umsetzung der Säureanhydride zu den erfindungsgemäßen Amiden wird in flüssiger Phase durchgeführt. Geeignete Reaktionsmedien sind beispielsweise Wasser, dipolare aprotische Lösungsmittel wie Acetonitril, N-Methylpyrrolidon, Dimethylformamid, Dimethylacetamid und dergleichen oder Gemische derselben. Die Reaktionstemperaturen liegen zwischen ca. 0 °C - 100 °C, wobei Temperaturen von 20 °C - 80 °C bevorzugt sind. Die Reaktionszeiten liegen zwischen 0.5 Stunden und 2 Tagen, vorzugsweise zwischen 1 Stunde und 36 Stunden.

Die Herstellung der Ester der allgemeinen Formel V erfolgt in bekannter Weise, z.B. nach den in R.A. Guilmette et al., J. Pharm. Sci. 68, 194 (1979) beschriebenen Verfahren.

Die Aminolyse der Ester erfolgt in flüssiger Phase, z.B. in einem geeigneten höhersiedenden Lösungsmittel wie Dimethylformamid, Dimethylacetamid oder Diemthylsulfoxid. Die Reaktionstemperaturen liegen bei etwa 20 °C - 200 °C, wobei Temperaturen von 100 °C - 180 °C bevorzugt sind. Die Reaktionszeiten liegen zwischen 2 Stunden und 2 Tagen, wobei Reaktionszeiten zwischen 4 Stunden und 36 Stunden bevorzugt sind.

Darüber hinaus können alle dem Fachmann bekannten Methoden zur Umwandlung von Carboxylgruppen in Amidgruppen zur Synthese der erfindungsgemäßen Komplexbildner der allgemeinen Formel I herangezogen werden, so z.B. die Methode nach Krejcarek und Tucker, Biochem. Biophys. Res. Commun. 77, 581 (1977) über gemischte Anhydride.

Die so erhaltenen Verbindungen der allgemeinen Formel I mit X in der Bedeutung eines Wasserstoffatoms stellen Komplexbildner dar. Sie können isoliert und gereinigt werden oder ohne Isolierung in Metallkomplexe der allgemeinen Formel I mit mindestens zwei der Substituenten X in der Bedeutung eines Metallionenäquivalents überführt werden.

Die Herstellung der erfindungsgemäßen Metallkomplexe erfolgt in der Weise, wie sie in den Patentschriften EP 71564, EP 130934 und DE-OS 34.01.052 offenbart worden ist, indem man das Metalloxid oder ein Metallsalz (beispielsweise das Nitrat, Acetat, Carbonat, Chlorid oder Sulfat) des Elements der Ordnungszahlen 21-29, 31, 32, 38, 39, 42-44, 49, 57-83 in Wasser und/oder einem niederen Alkohol (wie Methanol, Ethanol oder Isopropanol) löst oder suspendiert und mit der Lösung oder Suspension der äquivalenten Menge der Komplexbildenden Säure der allgemeinen Formel I mit X in der Bedeutung eines Wasserstoffatoms umsetzt und anschließend, falls gewünscht, vorhandene azide Wasserstoffatome von Säure-Gruppen durch Kationen anorganischer und/oder organischer Basen oder Aminosäuren substituiert.

Die Neutralisation erfolgt dabei mit Hilfe anorganischer Basen (z.B. Hydroxiden, Carbonaten oder Bicarbonaten) von z.B. Natrium, Kalium oder Lithium und/oder organischer Basen wie unter anderem

primärer, sekundärer und tertiärer Amine, wie z.B. Ethanolamin, Morpholin, Glucamin, N-Methyl- und N,N-Dimethylglucamin, sowie basischer Aminosäuren, wie z.B. Lysin, Arginin und Ornithin.

Zur Herstellung der neutralen Komplexverbindungen kann man beispielsweise den sauren Komplexsalzen in wäßriger Lösung oder Suspension soviel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie z.B. niederen Alkoholen (Methanol, Ethanol, Isopropanol etc.), niederen Ketonen (Aceton etc.), polaren Ethern (Tetrahydrofuran, Dioxan, 1,2-Dimethoxyethan etc.) auszufällen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen.

Enthalten die sauren Komplexverbindungen mehrere freie azide Gruppen, so ist es oft zweckmäßig, neutrale Mischsalze herzustellen, die sowohl anorganische als auch organische Kationen als Gegenionen enthalten.

Dies kann beispielsweise geschehen, indem man die komplexbildende Säure in wäßriger Suspension oder Lösung mit dem Oxid oder Salz des das Zentralion liefernden Elements und der Hälfte der zur Neutralisation benötigten Menge einer organischen Base umsetzt, das gebildete Komplexsalz isoliert, es gewünschtenfalls aufreinigt und dann zur vollständigen Neutralisation mit der benötigten Menge anorganischer Base versetzt. Die Reihenfolge der Basenzugabe kann auch umgekehrt werden.

Im Falle der Verwendung von Radioisotope enthaltenden Komplexverbindungen kann deren Herstellung nach den in "Radiotracers for Medical Applications", Volume I, CRC-Press, Boca Raton, Florida beschriebenen Methoden vorgenommen werden.

Die Herstellung der erfindungsgemäßen diagnostischen Mittel erfolgt ebenfalls in an sich bekannter Weise, indem man die erfindungsgemäßen Komplexverbindungen - gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie z.B. Tromethamin), geringe Zusätze von Komplexbildnern (wie z.B. Diethylentriaminpentaessigsäure) oder, falls erforderlich, Elektrolyte wie z.B. Natriumchlorid oder, falls erforderlich, Antioxidantien wie z.B. Ascorbinsäure.

Sind für die enterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoffen (z.B. Methylcellulose, Lactose, Mannit) und/oder Tensiden (z.B. Lecithine, Tweens$^{(R)}$, Myrj$^{(R)}$ und/oder Aromastoffen zur Geschmackskorrektur (z.B. ätherischen Ölen) gemischt.

Prinzipiell ist es auch möglich, die erfindungsgemäßen diagnostischen Mittel auch ohne Isolierung der Komplexsalze herzustellen. In jedem Fall muß besondere Sorgfalt darauf verwendet werden, die Chelatbildung so vorzunehmen, daß die erfindungsgemäßen Salze und Salzlösungen praktisch frei sind von nicht komplexierten toxisch wirkenden Metallionen.

Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexsalzes.

Die erfindungsgemäßen diagnostischen Mittel enthalten vorzugsweise 1 $\mu$Mol - 1 Mol/l des Komplexsalzes und werden in der Regel in Mengen von 0.001 - 5 mMol/kg dosiert. Sie sind zur enteralen und parenteralen Applikation bestimmt.

Die erfindungsgemäßen Komplexverbindungen kommen zur Anwendung

1.) für die NMR- und Röntgen-Diagnostik in Form ihrer Komplexe mit den Ionen der Elemente mit den Ordnungszahlen
21-29, 42, 44 und 57-83;

2.) für die Radiodiagnostik und -Therapie in Form ihrer Komplexe mit den Radioisotopen der Elemente mit den Ordnungszahlen
27, 29, 31, 32, 38, 39, 43, 49, 64, 70 und 77.

Die erfindungsgemäßen Mittel erfüllen die vielfältigen Voraussetzungen für die Eignung als Kontrastmittel für die Kernspintomographie. So sind sie hervorragend dazu geeignet, nach oraler oder parenteraler Applikation durch Erhöhung der Signalintensität das mit Hilfe des Kernspintomographen erhaltene Bild in seiner Aussagekraft zu verbessern. Die erfindungsgemäßen Komplexverbindungen können auch vorteilhaft als Shift-Reagenzien sowie zur Beeinflussung der magnetischen Eigenschaften von Atomkernen anderer Elemente wie z.B. $^{19}F$ und $^{31}P$ verwendet werden. Ferner zeigen sie hohe Wirksamkeit, die notwendig ist,

um den Körper mit möglichst geringen Mengen an Fremdstoffen zu belasten und die gut Verträglichkeit, die notwendig ist, um den nichtinvasiven Charakter der Üntersuchungen aufrechtzuerhalten.

Die gute Wasserlöslichkeit der erfindungsgemäßen Mittel erlaubt es hochkonzentrierte Lösungen herzustellen, damit die Volumenbelastung des Kreislaufs in vertretbaren Grenzen zu halten und die Verdünnung durch die Körperflüssigkeit auszugleichen, d.h. NMR-Diagnostika müssen 100 - 1000fach besser wasserlöslich sein als für die in-vitro-NMR-Spektroskopie. Weiterhin weisen die erfindungsgemäßen Mittel nicht nur eine hohe Stabilität in vitro auf, sondern auch eine überraschend hohe Stabilität in vivo, so daß eine Freigabe oder ein Austausch der in den Komplexen nicht kovalent gebundenen - an sich giftigen - Ionen innerhalb der Zeit, in der die neuen Kontrastmittel vollständig wieder ausgeschieden werden, nur äußerst langsam erfolgt.

Im allgemeinen werden die erfindungsgemäßen Mittel für die Anwendung als NMR-Diagnostika in Mengen von 0.001 - 5 mMol/kg, vorzugsweise 0.005 - 0.5 mMol/kg, dosiert. Details der Anwendung werden z.B. in H.J. Weinmann et al., Am. J. of Roentgenology 142, 619 (1984) diskutiert.

Besonders niedrige Dosierungen (unter 1 mg/kg) von organspezifischen NMR-Diagnostika sind zum Beispiel zum Nachweis von Tumoren und von Herzinfarkten einsetzbar.

Die erfindungsgemäßen Mittel sind aufgrund ihrer günstigen radioaktiven Eigenschaften und der guten Stabilität der in ihnen enthaltenen Komplexverbindungen auch als Radiodiagnostika geeignet.

Details ihrer Anwendung und Dosierung werden z.B. in "Radiotracers for Medical Applications", CRC-Press, Boca Raton, Florida beschrieben.

Die erfindungsgemäßen Mittel sind hervorragend als Röntgenkontrastmittel geeignet, wobei besonders hervorzuheben ist, daß sich mit ihnen keine Anzeichen der von den jodhaltigen Kontrastmitteln bekannten anaphylaxieartigen Reaktionen in biochemisch-pharmakologischen Üntersuchungen erkennen lassen. Besonders wertvoll sind sie wegen der günstigen Absorptionseigenschaften in Bereichen höherer Röhrenspannungen für digitale Substraktionstechniken.

Im allgemeinen werden die erfindungsgemäßen Mittel für die anwendung als Röntgenkontrastmittel in Analogie zu z.B. Meglumin-Diatrizoat in Mengen von 0.1 - 5 mMol/kg, vorzugsweise 0.25 - 1 mMol/kg, dosiert.

Details der Anwendung von Röntgenkontrastmitteln werden z.B. in Barke, Röntgenkontrastmittel, G. Thieme, Leipzig (1970) und P. Thurn, E. Bücheler "Einführung in die Röntgendiagnostik", G. Thieme, Stuttgart, New York (1977) diskutiert.

Insgesamt ist es somit gelungen, neue Komplexbildner, Metallkomplexe und Metallkomplexsalze zu synthetisieren, die neue Möglichkeiten in der diagnostischen und therapeutischen Medizin erschliessen. Vor allem die Entwicklung neuartiger bildgebender Verfahren in der medizinischen Diagnostik lässt diese Entwicklung wünschenswert erscheinen.

Die nachfolgenden Beispiele dienen zur Erläuterung des Erfindungsgegenstandes.

Beispiel 1

a) $N^6$-Carboxymethyl-$N^3$-ethoxycarbonylmethyl-$N^9$-3-oxapentamethylen-carbamoylmethyl-3,6,9-triazaundecandisäure

2.42 g (6 mMol) $N^3$-(2,6-Dioxomorpholinoethyl)-$N^6$-(ethoxycarbonylmethyl)-3,6-diazaoctandisäure werden in 30 ml Dimethylformamid suspendiert. Dann gibt man bei -5 °C 3.04 g (30 mMol) Triethylamin und 0.52 ml (6 mMol) Morpholin zu, läßt bei dieser Temperatur 2 h, darauf über Nacht bei Raumtemperatur rühren. Man engt die Lösung im Vakuum zur Trockne ein und verrührt den Rückstand mehrere Stunden mit 100 ml Diisopropylether. Man saugt ab und erhält nach Ümkristallisieren aus Ethanol 2.2 g (76 % der Theorie) eines weißen Pulvers vom Schmelzpunkt 130 °C (unter Aufschäumen).

| Analyse (bezogen auf wasserfreie Substanz): | | | |
|---|---|---|---|
| C 48.97 | H 6.99 | N 11.41 | (Ber.) |
| C 48.78 | H 7.15 | N 11.55 | (Gef.) |

b) $N^3$,$N^6$-Bis(carboxymethyl)-$N^9$-3-oxapentamethylen-carbamoyl-methyl-3,6,9-triazaundecandisäure

0.74 g (1.5 mMol) der unter a) erhaltenen Verbindung werden in 12 ml Wasser und 3 ml (15 mMol) 5n-Natronlauge gelöst. Man läßt 2 h bei Raumtemperatur rühren, bringt durch Zugabe von Amberlite® IR 120 auf $p_H$ 7, engt das Filtrat auf 10 ml ein, säuert es durch Zugabe von 8 ml Amberlite® IR 120 an, saugt ab und engt das Filtrat im Vakuum zur Trockne ein. Man erhält 720 mg (100 % der Theorie) eines weißen hydratwasserhaltigen Pulvers vom Schmelzpunkt 122 °C (unter Aufschäumen).

| Analyse (bezogen auf wasserfreie Substanz): | | | |
|---|---|---|---|
| C 46.75<br>C 46.52 | H 6.54<br>H 6.80 | N 12.11<br>N 12.02 | (Ber.)<br>(Gef.) |

c) Natriumsalz des Gadolinium(III)-Komplexes der $N^3$,$N^6$-Bis(carboxymethyl)-$N^9$-3-oxapentamethylen-carbamoylmethyl-3,6,9-triazaundecandisäure

720 mg (1.5 mMol) der unter b) erhaltenen Verbindung werden in 5 ml Wasser bei 50 °C mit 371 mg (0.75 mMol) Gadolinium(III)-carbonat, $Gd_2(CO_3)_3$, solange erwärmt, bis die Kohlendioxidentwicklung beendet ist. Dann bringt man die Lösung durch Zugabe von 0.1n-Natronlauge auf $p_H$ 7.2 und engt die Lösung im Vakuum zur Trockne ein. Nach dem Trocknen im Vakuum bei 50 °C erhält man 980 mg eines hydratwasserhaltigen weißen Pulvers mit einem oberhalb 300 °C liegenden Zersetzungspunkt.

| Analyse (bezogen auf wasserfreie Substanz): | | | | |
|---|---|---|---|---|
| C 33.85<br>C 33.71 | H 4.10<br>H 4.41 | N 8.77<br>N 8.50 | Gd 24.62<br>Gd 24.30 | (Ber.)<br>(Gef.) |

Beispiel 2

a) Gadolinium-III-komplex der $N^6$-carboxymethyl-$N^3$,$N^9$-bis (3-oxapentamethylen-carbamoylmethyl-3,6,9-triazaundecandisäure

Man löst 7.15 g (20 mMol) 1,5-Bis-(2.6-Dioxomorpholino )-3-aza-pentan-3-essigsäure in einer Mischung aus 5.23 ml (60 mMol) Morpholin und 55 ml Wasser. Nach 16 h versetzt man mit 6.69 g (20 mMol) Gadolinium(III)-acetat, gelöst in 80 ml Wasser, rührt über Nacht und gibt die Reaktionslösung über eine Säule mit 200 ml Anionenaustauscher IRA 410. Man eluiert mit 1 l Wasser und gibt das Eluat auf 80 ml Kationenaustauscher IRC 50. Man eluiert mit Wasser (1.5 l), engt das Eluat im Vakuum auf 200 ml ein und rührt die Lösung mit 10 ml Anionenaustauscher IRA 410 für 30 min, saugt ab und rührt das Filtrat weitere 30 min mit 10 ml Kationenaustauscher IRC 50, saugt ab und dampft im Vakuum ein. Der Rückstand wird pulverisiert und im Vakuum bei 70 °C getrocknet. Man erhält 8.90 g der Titelverbindung als weißes Pulver vom Schmelzpunkt oberhalb 300 °C.

| Analyse (bezogen auf wasserfreie Substanz): | | | | |
|---|---|---|---|---|
| C 38.53<br>C 38.31 | H 5.00<br>H 5.07 | Gd 22.93<br>Gd 23.19 | N 10.21<br>N 10.08 | (Ber.)<br>(Gef.) |

b) Gadolinium-III-komplex der $N^6$-Carboxymethyl-$N^3$,$N^9$-bis(tetramethylen-carbamoylmethyl)-3,6,9-triazaundecandisäure durch Reaktion mit Pyrrolidin.

Eigenschaften: weißes Pulver mit einem Schmelzpunkt oberhalb 300 °C.

| Analyse (bezogen auf wasserfreie Substanz): | | | | |
|---|---|---|---|---|
| C 40.42<br>C 40.24 | H 5.24<br>H 5.05 | Gd 24.05<br>Gd 24.05 | N 10.71<br>N 10.75 | (Ber.)<br>(Gef.) |

c) Gadolinium-III-komplex der $N^6$-Carboxymethyl-$N^3$,$N^9$-bis(pentamethylen-carbamoylmethyl)-3,6,9-triazaundecandisäure durch Reaktion mit Pyrrolidin.

Eigenschaften: weißes Pulver mit einem Schmelzpunkt oberhalb 300 °C.

| Analyse (bezogen auf wasserfreie Substanz): | | | | |
|---|---|---|---|---|
| C 42.28<br>C 42.01 | H 5.62<br>H 5.57 | Gd 23.06<br>Gd 23.24 | N 10.27<br>N 10.31 | (Ber.)<br>(Gef.) |

d) Gadolinium-III-komplex der $N^6$-Carboxymethyl-$N^3$,$N^9$-bis[N,N-3-(2-hydroxyethyl)-3-azapentamethylen-carbamoylmethyl]-3,6,9-triazaundecandisäure durch Reaktion mit N-(2-Hydroxyethyl)-piperazin.
Eigenschaften: weißes Pulver mit einem Schmelzpunkt oberhalb 300 °C.

| Analyse (bezogen auf wasserfreie Substanz): | | | | |
|---|---|---|---|---|
| C 40.46<br>C 40.53 | H 5.75<br>H 5.91 | Gd 20.37<br>Gd 20.18 | N 12.70<br>N 12.55 | (Ber.)<br>(Gef.) |

e) Gadolinium-III-Komplex der $N^6$-Carboxymethyl-$N^3$,$N^9$-bis[N,N-(1-hydroxymethyl)-pentamethylen-carbamoylmethyl]-3,6,9-triazaundecandisäure durch Reaktion mit 2-Hydroxymethylpiperidin.
Eigenschaften: weißes Pulver mit einem Schmelzpunkt oberhalb 300 °C.

| Analyse (bezogen auf wasserfreie Substanz): | | | | |
|---|---|---|---|---|
| C 42.09<br>C 42.01 | H 5.71<br>H 5.62 | Gd 21.20<br>Gd 21.45 | N 9.44<br>N 9.53 | (Ber.)<br>(Gef.) |

## Beispiel 3

Natriumsalz des Gadolinium-III-komplexes der $N^3$,$N^6$-bis(carboxymethyl-$N^9$-(3-oxapentamethylen)-carbamoylmethyl-3,6,9-triazaundecandisäure

In einem 1 l-Autoklaven werden 42.1 g (0.1 Mol) $N^3$,$N^6$-bis(carboxymethyl)-$N^9$-ethoxycarbonylmethyl-3,6,9-triazaundecandisäure (hergestellt nach J. Pharm. Sci. 68 (1979), 194) mit 87.12 g (1 Mol) Morpholin 30 h auf 110 °C erhitzt. Nach dem Abkühlen löst man in 200 ml Methanol und engt im Vakuum zur Trockne ein. Der Rückstand wird in 500 ml Wasser gelöst und mit 41.2 g (0.1 Mol) Gadolinium(III)-acetat (Wassergehalt 18.9 %) versetzt. Man läßt die Lösung 2 h bei Raumtemperatur rühren, entsalzt sie über Amberlite® IRA 410 und dann über Amberlite® IR 120 und bringt das Filtrat durch Zugabe von 2n-Natronlauge auf $p_H$ 7.2. Nach dem Gefriertrocknen erhält man 68.5 g (96.3 % der Theorie) des gewünschten Salzes als Tetrahydrat in Form eines weißen Pulvers vom Schmelzpunkt 275 °C (unter Aufschäumen).

| Analyse (bezogen auf wasserfreie Substanz): | | | | |
|---|---|---|---|---|
| C 33.85<br>C 33.93 | H 4.10<br>H 4.25 | N 8.77<br>N 8.91 | Gd 24.62<br>Gd 24.48 | (Ber.)<br>(Gef.) |

## Beispiel 4

Mn(II)-Komplex vom trans-1,2-Diamino-N,N′-bis(carboxymethyl)-N,N′-bis(3-oxapentamethylen-carbamoylmethyl)-cyclohexan

9.3 g (30 mMol) 4,4′-(trans-1,2-Cyclohexandiyl)-bis-(2,6-morpholindion), hergestellt nach DOS DE 3.324.236 werden in 50 ml Wasser suspendiert und mit 5.23 ml (60 mMol) Morpholin versetzt. Nach 16stündigem Rühren bei Raumtemperatur versetzt man die klare Reaktionslösung mit 3.45 g (30 mMol) Mangan(II)-carbonat, $MnCO_3$, unter Stickstoffbegasung. Nach Beendigung der $CO_2$-Entwicklung wird die Lösung gefriergetrocknet. Man erhält 18.4 g der Titelverbindung als hydratwasserhaltiges braunes Pulver mit einem oberhalb 300 °C liegenden Zersetzungspunkt.

| Analyse (bezogen auf wasserfreie Substanz): | | | | |
|---|---|---|---|---|
| C 49.16<br>C 49.33 | H 6.38<br>H 6.52 | N 10.42<br>N 10.20 | Mn 10.22<br>Mn 10.50 | (Ber.)<br>(Gef.) |

**BEISPIEL 5**

Gadolinium-III-Komplex von 3,6,9-Tris-(carboxymethyl)-3,6,9-triazaundecandisäure-N,N'-bis(2-carbamoylpentamethylen)-diamid

Zu einer Lösung von 8,09 g (60 mMol; 95%ig) Piperidin-3-carbonsäureamid in 80 ml Wasser gibt man unter Rühren 7,15 g (20 mMol) 1,5-Bis-(2,6-dioxomorpholino)-3-aza-pentan-3-essigsäure, rührt 20 Stunden bei Raumtemperatur und versetzt dann mit 6,69 g (20 mMol) Gadolinium-III-acetat gelöst in 80 ml Wasser. Nach weiteren 24 Stunden gibt man die Reaktionslösung über eine Säule mit 20 ml Anionenaustauscher IRA 410, eluiert mit 1 Liter Wasser und gibt das Eluat auf 80 mol eines Kationenaustauschers IRC 50. Man engt das Eluat auf ca. 200 ml im Vakuum ein, rührt die Lösung mit 10 ml IRA 410, filtriert, rührt mit 10 ml IRC 50, filtriert und dampft die Lösung im Vakuum ein.
Man erhält 11,2 g der Titelverbindung als weißes Pulver, Schmelzpunkt oberhalb 300°, Wassergehalt: 3,45%.

| Analyse (bezogen auf wasserfreie Substanz): | | | | |
|---|---|---|---|---|
| Ber.: | C 40,67 | H 5,25 | Gd 20,48 | N 12,77 |
| Gef.: | C 40,74 | H 5,44 | Gd 20,33 | N 12,78 |

**BEISPIEL 6**

Gadolinium-III-Komplex von 3,6,9-Tris-(carboxymethyl)-3,6,9-triazaundecandisäure-N,N'-bis-(3-carbamoylpentamethylen)-diamid

Setzt man an Stelle von Piperidin-3-carbonsäureamid des Beispiels 5 60 mMol Piperidin-4-carbonsäureamid ein, so erhält man 8,08 g des Gadoliniumkomplexes von 3,6,9-tris-(carboxymethyl)-3,6,9-triazaundecandisäure-N,N'-bis-(3-carbamoylpentamethylen)-diamid. Schmelzpunkt oberhalb von 300°C; Wassergehalt: 3,60%

| Analyse (bezogen auf wasserfreie Substanz): | | | | |
|---|---|---|---|---|
| Ber.: | C 40,67 | H 5,25 | Gd 20,48 | N 12,77 |
| Gef.: | C 40,62 | H 5,80 | Gd 20,39 | N 12,59 |

Beispiel 7

N-Methylglucaminsalz des Gadolinium(III)-Komplexes der $N^3$,$N^6$-Bis(carboxymethyl)-$N^9$-diethylcarbamoylmethyl-3,6,9-triazaundecandisäure

In einem 1 l-Autoklaven werden 42.1 g (0.1 Mol) $N^3$,$N^6$-Bis(carboxymethyl)-$N^9$-ethoxycarbonylmethyl-3,6,9-triazaundecandisäure (hergestellt nach J. Pharm. Sci. 68 (1979), 194) mit 73.1 g (1 Mol) Diethylamin 30 h auf 110°C erhitzt. Nach dem Abkühlen löst man 200 ml Methanol und engt im Vakuum zur Trockne ein. Man erhält 73.4 g des Tetraethylammoniumsalzes der $N^3$,$N^6$-Bis(carboxymethyl)-$N^9$-diethylcarbamoylmethyl-3,6,9-triazaundecandisäure. Zur Lösung des Salzes in 500 ml Wasser gibt man 41.2 g (0.1 Mol) Gadolinium(III)-acetat (Wassergehalt 18.9 %) und läßt 2 h bei Raumtemperatur rühren. Dann entsalzt man die Lösung über Ionenaustauscher und bringt sie durch Zugabe von N-Methylglucamin auf $p_H$ 7.0. Nach dem Gefriertrocknen erhält man 74.3 g des gewünschten Salzes als hydratwasserhaltiges weißes Pulver mit einem oberhalb 300°C liegenden Zersetzungspunkt.

| Analyse (bezogen auf wasserfreie Substanz): | | | | |
|---|---|---|---|---|
| C 37.68 | H 5.69 | N 8.79 | Gd 19.73 | (Ber.) |
| C 37.44 | H 5.80 | N 8.71 | Gd 19.91 | (Gef.) |

Beispiel 8

Gadolinium(III)-Komplex der $N^6$-Carboxymethyl-$N^3$,$N^9$-bis(dimethylcarbamoylmethyl)-3,6,9-triazaundecandisäure

In einem 1 l-Autoklaven werden 44.9 g (0.1 Mol) 6-Carboxymethyl-3,9-bis(ethoxycarbonylmethyl)-3,6,9-triazaundecandisäure, hergestellt nach J. Pharm. Sci. 68 (1979), 194, mit 100 ml Ethanol und 45 g (1 Mol) Dimethylamin 30 h auf 100 °C erhitzt. Nach dem Abkühlen engt man im Vakuum zur Trockne ein und löst den Rückstand in 200 ml Wasser. Man versetzt mit einer Lösung von 41.20 g Gadolinium(III)-acetat (0.1 Mol, Wassergehalt 18.87 %) und läßt 2 h bei Raumtemperatur rühren. Dann wird die Lösung über Ionenaustauscher entsalzt und gefriergetrocknet. Man erhält 58.0 g (94 % der Theorie) vom Gadolinium(III)-Komplex der $N^6$-Carboxymethyl-$N^3$,$N^9$-bis(dimethylcarbamoylmethyl)-3,6,9-triazaundecandisäure als Monohydrat mit einem oberhalb 305 °C liegenden Zersetzungspunkt.

| Analyse (bezogen auf wasserfreie Substanz): | | | | |
|---|---|---|---|---|
| C 35.93<br>C 36.15 | H 5.03<br>H 5.21 | N 11.64<br>N 11.77 | Gd 26.13<br>Gd 26.02 | (Ber.)<br>(Gef.) |

Beispiel 9

Natriumsalz des Dysprosium-(III)-Komplexes der $N^3$,$N^6$-Bis(carboxymethyl)-$N^9$-phenylcarbamoylmethyl-3,6,9-triazaundecandisäure

a) $N^3$,$N^6$-Bis(carboxymethyl)-$N^9$-phenylcarbamoylmethyl-3,6,9-triazaundecandisäure

In einem 1 l-Autoklaven werden 42.1 g (0.1 Mol) $N^3$,$N^6$-Bis(carboxymethyl)-$N^9$-ethoxycarbonylmethyl-3,6,9-triazaundecandisäure mit 93.1 g (1 Mol) Anilin 30 h auf 110 °C erhitzt. Nach dem Abkühlen löst man den Inhalt in 800 ml Methanol und engt die Lösung im Vakuum zur Trockne ein. Der Rückstand wird mit 800 ml Wasser versetzt und das überschüssige Anilin mit Methylenchlorid erschöpfend extrahiert. Die wäßrige Phase wird nach Andestillieren über 100 ml Amberlite® IR 120 gegeben. Das saure Eluat wird eingeengt und der Rückstand im Vakuum bei 50 °C getrocknet. Man erhält 35.1 g (75 % der Theorie) als weißes Pulver mit einem Schmelzpunkt von 125 °C.

| Analyse (bezogen auf wasserfreie Substanz): | | | |
|---|---|---|---|
| C 51.27<br>C 51.44 | H 6.03<br>H 6.21 | N 11.96<br>N 12.03 | (Ber.)<br>(Gef.) |

b) 3.9 g (8.3 mMol) der unter a) erhaltenen Verbindung werden in 50 ml Wasser suspendiert und mit 2.82 g (8.3 mMol) Dysprosium-(III)-acetat versetzt. Man läßt 2 h bei 60 °C rühren und entfernt nach Abkühlen auf Raumtemperatur die Acetationen über einen Austauscher. Man bringt die Lösung durch Zugabe von 0.1n-Natronlauge auf $p_H$ 7. Nach dem Einengen zur Trockne erhält man in qualitativer Ausbeute das gewünschte Komplexsalz als hydratwasserhaltiges gelbliches Pulver.

| Analyse (bezogen auf wasserfreie Substanz): | | | | |
|---|---|---|---|---|
| C 36.96<br>C 36.85 | H 3.72<br>H 3.92 | N 8.62<br>N 8.81 | Dy 25.00<br>Dy 25.23 | (Ber.)<br>(Gef.) |

Beispiel 10

Gadolinium-(III)-Komplex der $N^6$-Carboxymethyl-$N^3$,$N^9$-bis(N-methyl-N-n-propylcarbamoylmethyl)-3,6,9-triazaundecandisäure

Man löst 7.15 g (20 mMol) 1,5-Bis-(2,6-dioxomorpholino)-3-azapentan-3-essigsäure in einer Mischung

aus 4.39 g (60 mMol) N-Methyl-n-propylamin und 60 ml Wasser. Nach 20 h versetzt man mit 6.69 g (20 mMol) Gadolinium-(III)-acetat, gelöst in 80 ml Wasser. Nach weiteren 16 h filtriert man die Lösung und läßt das Filtrat durch eine Säule mit 200 ml Anionenaustauscher IRA 410 laufen. Man eluiert mit 1 l Wasser und gibt das Eluat auf 80 ml Kationenaustauscher IRC 50. Das Eluat wird dann auf 150 ml im Vakuum eingeengt und die Lösung 1 h mit 10 ml Anionenaustauscher IRA 410 gerührt und filtriert. Das Filtrat rührt man noch 1 h mit 10 ml Kationenaustauscher IRC 50, filtriert und dampft die Lösung im Vakuum ein. Der Rückstand wird pulverisiert und bei 70 ° C im Vakuum getrocknet. Man erhält 8.73 g der Titelverbindung als weißes Pulver vom Schmelzpunkt oberhalb 300 ° C.

| Analyse (bezogen auf wasserfreie Substanz): | | | | |
|---|---|---|---|---|
| C 40.17<br>C 39.98 | H 5.82<br>H 5.99 | Gd 23.90<br>Gd 24.20 | N 10.65<br>N 10.66 | (Ber.)<br>(Gef.) |

Wenn man nach der vorstehenden Vorschrift verfährt und N-Methyl-n-propylamin jeweils durch ein anderes Amin ersetzt, erhält man die folgenden Komplexverbindungen:

a) Gadolinium-III-Komplex der $N^6$-Carboxymethyl-$N^3$,$N^9$-bis(diethyl-carbamoylmethyl)-3,6,9-triazaundecandisäure durch Reaktion mit Diethylamin.
Eigenschaften: weißes Pulver mit einem Schmelzpunkt oberhalb 300 ° C.

| Analyse (bezogen auf wasserfreie Substanz): | | | | |
|---|---|---|---|---|
| C 40.17<br>C 40.06 | H 5.82<br>H 6.03 | Gd 23.90<br>Gd 24.30 | N 10.65<br>N 10.51 | (Ber.)<br>(Gef.) |

b) Gadolinium-III-Komplex der $N^6$-Carboxymethyl-$N^3$,$N^9$-bis(dimethyl-carbamoylmethyl)-3,6,9-triazaundecandisäure durch Reaktion mit Dimethylamin.
Eigenschaften: weißes Pulver mit einem Schmelzpunkt oberhalb 300 ° C.

| Analyse (bezogen auf wasserfreie Substanz): | | | | |
|---|---|---|---|---|
| C 35.93<br>C 35.69 | H 5.03<br>H 4.97 | Gd 26.13<br>Gd 26.25 | N 11.64<br>N 11.51 | (Ber.)<br>(Gef.) |

Beispiel 11

Mn-(II)-Komplex vom trans-1,2-Diamino-N,N'-bis(carboxymethyl)-N,N'-bis(dimethylcarbamoylmethyl)-cyclohexan.

9.3 g (30 mMol) 4,4'-(trans-1,2-Cyclohexandiyl)-bis-2,6-morpholindion), hergestellt nach DOS DE 3.324.236, werden in 50 ml Wasser suspendiert und mit 4.5 g (100 mMol) Dimethylamin (wäßriger Lösung) versetzt. Man läßt 16 h bei Raumtemperatur rühren und engt die Lösung im Vakuum zur Trockne ein. Der Rückstand des Dimethylammoniumsalzes wird in 100 ml Wasser gelöst und unter Stickstoffbegasung mit 3.45 g (30 mMol) Mangan-(II)-carbonat, $MnCO_3$, versetzt. Nach Beendigung der $CO_2$-Entwicklung wird die Lösung gefriergetrocknet. Man erhält 15.2 g der Titelverbindung als hydratwasserhaltiges braunes Pulver mit einem oberhalb 300 ° C liegenden Zersetzungspunkt.

| Analyse (bezogen auf wasserfreie Substanz): | | | | |
|---|---|---|---|---|
| C 47.68<br>C 47.80 | H 6.67<br>H 6.83 | N 12.36<br>N 12.39 | Mn 12.12<br>Mn 12.31 | (Ber.)<br>(Gef.) |

**Patentansprüche**

1. Physiologisch verträgliche Verbindungen der allgemeinen Formel I

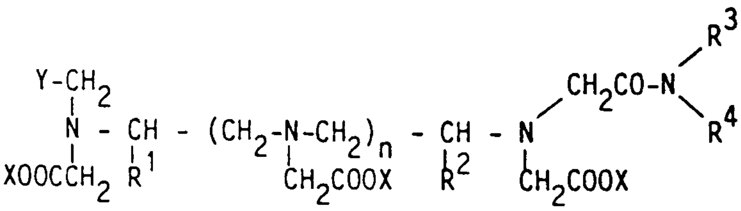 (I),

worin
n die Ziffern 0, 1 oder 2,
$R^1$ und $R^2$ Wasserstoffatome, niedere Alkylgruppen, Phenylgruppen, Benzylgruppen oder, wenn n für die Ziffer 0 steht, auch gemeinsam eine Trimethylen- oder eine Tetramethylengruppe,
$R^3$ ein gesättigter, ungesättigter, gerad- oder verzweigtkettiger oder cyclischer aliphatischer Kohlenwasserstoffrest mit bis zu 16 C-Atomen, oder, wenn $R^4$ ein Wasserstoffatom ist, eine Cycloalkyl- oder eine gegebenenfalls durch eine oder mehrere Di-$C_1$-$C_6$-alkylamino- oder durch eine oder mehrere $C_1$-$C_6$-Alkoxygruppen substituierte Aryl- oder Aralkylgruppe,
$R^4$ ein Wasserstoffatom, ein gesättigter, ungesättigter, gerad- oder verzweigtkettiger oder cyclischer Kohlenwasserstoffrest mit bis zu 16 C-Atomen,
mit der Maßgabe, daß - wenn $R^1$ und $R^2$ für Wasserstoffatome stehen - $R^3$ und $R^4$ nicht gleichzeitig für gesättigte, ungesättigte, gerad- oder verzweigtkettige aliphatische Kohlenwasserstoffreste mit bis zu 16 C-Atomen stehen, oder
$R^3$ und $R^4$ gemeinsam einen gesättigten oder ungesättigten, gegebenenfalls durch einen oder mehrere $C_1$-$C_6$-Alkyl-, $C_1$-$C_5$-Hydroxyalkyl-, einen gegebenenfalls hydroxylierten oder $C_1$-$C_6$-alkoxylierten $C_2$-$C_6$-Acyl-, Hydroxy-, Carbamoyl-, Carbamoyl-substituierten $C_1$-$C_6$-Alkyl-, am Carbamoyl-Stickstoff durch einen oder Zwei $C_1$-$C_6$-Alkylrest(e) - die auch einen gegebenenfalls ein Sauerstoffatom enthaltenen Ring bilden können - substituierten Carbamoyl-, oder einen $C_1$-$C_6$-Acylamino- oder $C_1$-$C_6$-Alkylaminorest substituierten 5- oder 6-Ring, der gegebenenfalls ein weiteres Stickstoff-, Sauerstoff-, Schwefelatom oder eine Carbonylgruppe enthält,
X ein Wasserstoffatom und/oder ein Metallionenäquivalent,
Y eine COOX- oder

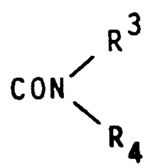

- Gruppe
bedeuten,
sowie deren Salze mit organischen und/oder anorganischen Basen.

2. Physiologisch verträgliche Verbindungen gemäß Anspruch 1, dadurch gekennzeichnet, daß X Wasserstoffatome darstellt.

3. Physiologisch verträglich Verbindungen gemäß Anspruch 1, dadurch gekennzeichnet, daß mindestens zwei der Substituenten X Metallionenäquivalente mindestens eines Elements der Ordnungszahlen 21-29, 42, 44 oder 57-83 sind.

4. Physiologisch verträglich Verbindungen gemäß Anspruch 1, dadurch gekennzeichnet, daß mindestens zwei der Substituenten X Metallionenäquivalente eines Radionuklids mindestens eines Elements der Ordnungszahlen 27, 29, 31, 32, 38, 39, 43, 49, 62, 64, 70 oder 77 sind.

5. Physiologisch verträglich Verbindungen gemäß Anspruch 1, dadurch gekennzeichnet, daß Y für eine COOX-Gruppe steht.

6. Physiologisch verträgliche Verbindungen gemäß Anspruch 1, dadurch gekennzeichnet, daß Y für eine

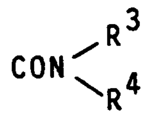

-Gruppe steht.

7. Diagnostische Mittel enthaltend mindestens eine physiologisch verträgliche Verbindung nach Anspruch 1 bis 6, gegebenenfalls mit den in der Galenik üblichen Zusätzen.

8. Verwendung von mindestens einer physiologisch verträglichen Verbindung gemäß Anspruch 3, 5 und 6 für die Herstellung von Mitteln für die NMR- oder Röntgen-Diagnostik.

9. Verwendung von mindestens einer physiologisch verträglichen Verbindung gemäß Anspruch 4 bis 6 für die Herstellung von Mitteln für die Radio-Diagnostik und -Therapie.

10. Verfahren zur Herstellung physiologisch verträglicher Komplexverbindungen der allgemeinen Formel I gemäss Anspruch 1, dadurch gekennzeichnet, dass man in an sich bekannter Weise
a)zur Herstellung von Verbindungen der allgemeinen Formel I, in denen Y eine

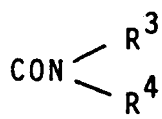

- Gruppe bedeutet, eine Verbindung der allgemeinen Formel II

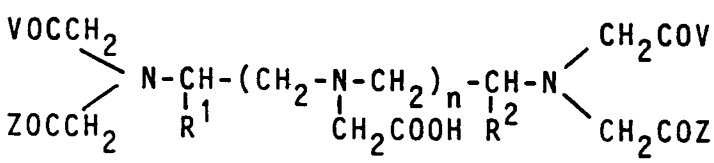

(II)

worin
$R^1$, $R^2$ und n die oben genannte Bedeutung haben und V und Z gemeinsam ein Sauerstoffatom oder V eine Hydroxygruppe und Z die Gruppierung $OR^5$ darstellen,
mit einem Amin der allgemeinen Formel III

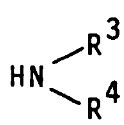

(III),

worin
$R^3$ und $R^4$ die oben genannte Bedeutung haben, umsetzt,
oder
b) zur Herstellung von Verbindungen der allgemeinen Formel I, in denen Y eine COOH-Gruppe bedeutet, eine Verbindungen der allgemeinen Formel VI

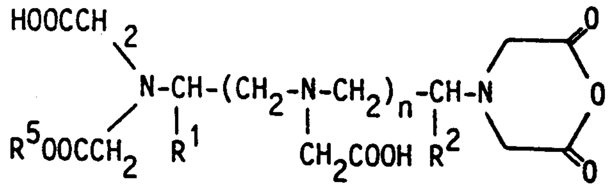

(VI),

oder V.

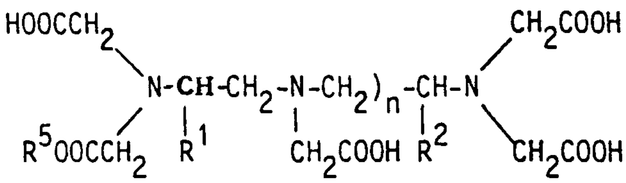

(V),

worin
$R^5$ einen $C_1$-$C_6$-Alkylrest darstellt und $R^1$, $R^2$ und n die in Anspruch 1 genannten Bedeutungen haben, mit einem Amin der allgemeinen Formel III

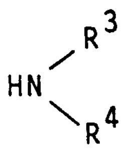

(III),

umsetzt und gegebenenfalls noch vorhandene Estergruppen verseift,
die so erhaltenen Säuren der allgemeinen Formel I mit X in der Bedeutung eines Wasserstoffatoms gewünschtenfalls in an sich bekannter Weise mit mindestens einem Metalloxid oder Metallsalz eines Elements der Ordnungszahlen 21-29, 31, 32, 38, 39, 42-44, 49, 57-83 umsetzt und anschließend,
falls gewünscht, vorhandene azide Wasserstoffatome durch Kationen anorganischer und/oder organischer Basen oder Aminosäuren substituiert.

**11.** Verfahren zur Herstellung der diagnostischen Mittel gemäß Anspruch 7 dadurch gekennzeichnet, daß man die in Wasser oder physiologischer Salzlösung gelöste oder suspendierte Komplexverbindung, gegebenenfalls mit den in der Galenik üblichen Zusätzen in eine für die enterale oder parenterale Applikation geeignete Form bringt.

**12.** $N^3$-(2,6-Dioxomorpholinoethyl-$N^6$-(ethoxycarbonylmethyl)-3,6-diazaoctandisäure.

**13.** N-Methylglucaminsalz des Gadolinium(III)-Komplexes der $N^3$,$N^6$-Bis(carboxymethyl)-$N^9$-diethylcarbamoylmethyl-3,6,9-triazaundecandisäure.
Gadolinium(III)-Komplex der $N^6$-Carboxymethyl-$N^3$,$N^9$-bis(dimethylcarbamoylmethyl)-3,6,9-triazaundecandisäure.
Natriumsalz des Dysprosium(III)-Komplexes der $N^3$,$N^6$-Bis(carboxymethyl)-$N^9$-phenylcarbamoylmethyl-3,6,9-triazaundecandisäure.
Gadolinium(III)-Komplex der $N^6$-Carboxymethyl-$N^3$,$N^9$-bis(N-methyl-N-n-propylcarbamoylmethyl)-3,6,9-triazaundecandisäure.
Gadolinium(III)-Komplex der $N^6$-Carboxymethyl-$N^3$,$N^9$-bis(diethyl-carbamoylmethyl)-3,6,9-triazaundecandisäure.
Gadolinium(III)-Komplex der $N^6$-Carboxymethyl-$N^3$,$N^9$-bis(dimethyl-carbamoylmethyl)-3,6,9-triazaundecandisäure.
Mangan(II)-Komplex vom trans-1,2-Diamino-N,N'-bis(carboxymethyl)-N,N'-bis-(dimethylcarbamoylmethyl)-cyclohexan.

**14.** $N^6$-Carboxymethyl-$N^3$-ethoxycarbonylmethyl-$N^9$-3-oxapentamethylen-carbamoylmethyl-3,6,9-

triazaundecandisäure
$N^3$,$N^6$-Bis(carboxymethyl)-$N^9$-3-oxapentamethylen-carbamoyl-3,6,9-triazaundecandisäure
Natriumsalz des Gadolinium-(III)-Komplexes der $N^3$,$N^6$-Bis-(carboxymethyl)-$N^9$-3-oxapentamethylen-carbamoylmethyl-3,6,9-triazaundecandisäure
Gadolinium-III-Komplex der $N^6$-Carboxymethyl-$N^3$,$N^9$-bis-(3-oxapentamethylen-carbamoylmethyl)-3,6,9-triazaundecandisäure
Gadolinium-III-Komplex der $N^6$-Carboxymethyl-$N^3$,$N^9$-bis-(tetramethylen-carbamoylmethyl)-3,6,9-triazaundecandisäure
Gadolinium-III-Komplex der $N^6$-Carboxymethyl-$N^3$,$N^9$-bis-(pentamethylen-carbamoylmethyl)-3,6,9-triazaundecandisäure
Gadolinium-III-Komplex der $N^6$-Carboxymethyl-$N^3$,$N^9$-bis-[N,N-3-(2-hydroxyethyl)-3-azapentamethylen-carbamoylmethyl]-3,6,9-triazaundecandisäure
Gadolinium-III-Komplex der $N^6$-Carboxymethyl-$N^3$,$N^9$-bis-[N,N-(1-hydroxymethyl)-pentamethylen-carbamoylmethyl]-3,6,9-triazaundecandisäure
Natriumsalz des Gadolinium-III-Komplexes der $N^3$,$N^6$-bis-(carboxymethyl)-$N^9$-(3-oxapentamethylen)-carbamoylmethyl-3,6,9-triazaundecandisäure
Mn(II)-Komplex vom trans-1,2-Diamino-N,N'-bis-(carboxymethyl)-N,N'-bis-(3-oxapentamethylen-carbamoylmethyl)-cyclohexan
Gadolinium-III-Komplex von 3,6,9-Tris-(carboxymethyl)-3,6,9-triazaundecandisäure-N,N'-bis-(2-carbamoylpentamethylen)-diamid
Gadolinium-III-Komplex von 3,6,9-Tris-(carboxymethyl)-3,6,9-triazaundecandisäure-N,N'-bis-(3-carbamoylpentamethylen)-diamid.

## Claims

**1.** Physiologically acceptable compounds of the general formula I

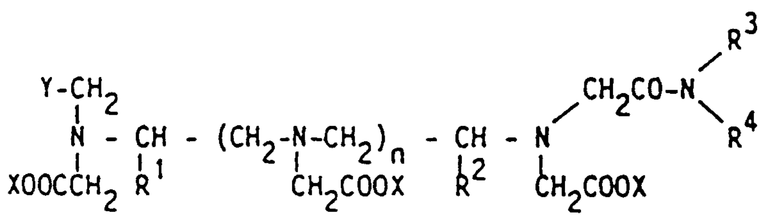

(I)

wherein
n represents the number 0, 1 or 2,
$R^1$ and $R^2$ represent hydrogen atoms, lower alkyl groups, phenyl groups or benzyl groups or, when n represents the number 0, $R^1$ and $R^2$ together represent a trimethylene or a tetramethylene group,
$R^3$ represents a saturated, unsaturated, straight- or branched-chained or cyclic aliphatic hydrocarbon radical having up to 16 carbon atoms or, when $R^4$ is a hydrogen atom, $R^3$ represents a cycloalkyl group or an aryl or aralkyl group optionally substituted by one or more di-$C_1$-$C_6$-alkylamino groups or by one or more $C_1$-$C_6$-alkoxy groups,
$R^4$ represents a hydrogen atom, a saturated, unsaturated, straight- or branched-chained or cyclic hydrocarbon radical having up to 16 carbon atoms,
with the proviso that, when $R^1$ and $R^2$ represent hydrogen atoms, $R^3$ and $R^4$ do not simultaneously represent saturated, unsaturated, straight- or branched-chained aliphatic hydrocarbon radicals having up to 16 carbon atoms,
or
$R^3$ and $R^4$ together represent a saturated or unsaturated 5- or 6-membered ring optionally substituted by one or more $C_1$-$C_6$-alkyl radicals, $C_1$-$C_5$-hydroxyalkyl radicals, optionally hydroxylated or $C_1$-$C_6$-alkoxylated $C_2$-$C_6$-acyl radicals, hydroxy groups, carbamoyl radicals, carbamoyl-substituted $C_1$-$C_6$-alkyl radicals, carbamoyl radicals substituted at the carbamoyl nitrogen by one or two $C_1$-$C_6$-alkyl radical(s) - which can also form a ring optionally containing an oxygen atom - or by a $C_1$-$C_6$-acylamino or $C_1$-$C_6$-alkylamino radical, the 5- or 6-membered ring optionally containing a further nitrogen, oxygen or sulphur atom or a carbonyl group,
X represents a hydrogen atom and/or a metal ion equivalent, and

Y represents a COOX or

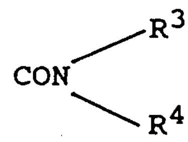

group, as well as their salts with organic and/or inorganic bases.

**2.** Physiologically acceptable compounds according to claim 1, characterised in that X represents hydrogen atoms.

**3.** Physiologically acceptable compounds according to claim 1, characterised in that at least two of the X substituents are metal ion equivalents of at least one element of atomic numbers 21-29, 42, 44 or 57-83.

**4.** Physiologically acceptable compounds according to claim 1, characterised in that at least two of the X substituents are metal ion equivalents of a radionuclide of at least one element of atomic numbers 27, 29, 31, 32, 38, 39, 43, 49, 62, 64, 70 or 77.

**5.** Physiologically acceptable compounds according to claim 1, characterised in that Y represents a COOX group.

**6.** Physiologically acceptable compounds according to claim 1, characterised in that Y represents a

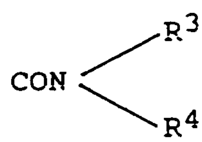

group.

**7.** Diagnostic media containing at least one physiologically acceptable compound according to any one of claims 1 to 6, optionally together with the additives customary in galenic pharmacy.

**8.** Use of at least one physiologically acceptable compound according to any one of claims 3, 5 and 6 for the production of media for NMR or X-ray diagnostics.

**9.** Use of at least one physiologically acceptable compound according to any one of claims 4 to 6 for the production of media for radiodiagnostics and radiotherapy.

**10.** Process for the preparation of physiologically acceptable complex compounds of the general formula I according to claim 1, characterised in that in a manner known per se

(a) for the preparation of compounds of the general formula I wherein Y represents a

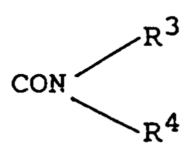

group, a compound of the general formula II

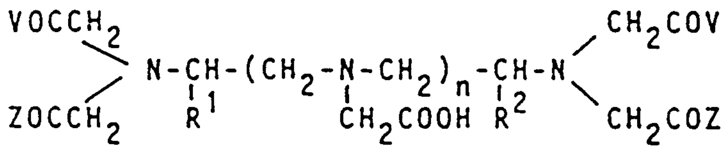

(II),

wherein
$R^1$, $R^2$ and n have the meanings given above and
V and Z together represent an oxygen atom or
V represents a hydroxy group and Z represents the grouping $OR^5$,
is reacted with an amine of the general formula III

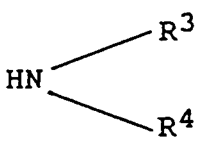

(III),

wherein
$R^3$ and $R^4$ have the meanings given above,
or
(b) for the preparation of compounds of the general formula I wherein Y represents a COOH group, a compound of the general formula VI

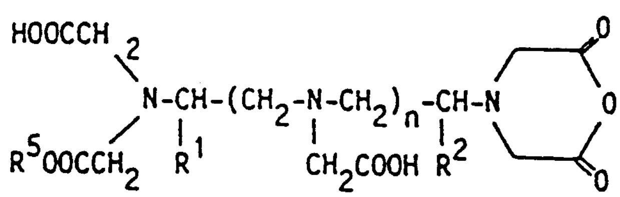

(VI)

or V

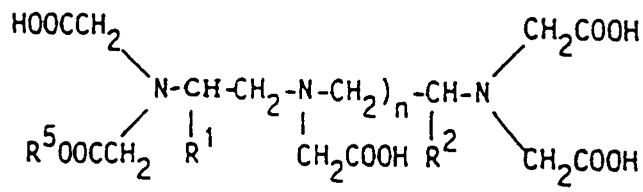

(V),

wherein
$R^5$ represents a $C_1$-$C_6$-alkyl radical and $R^1$, $R^2$ and n have the meanings given in claim 1,
is reacted with an amine of the general formula III

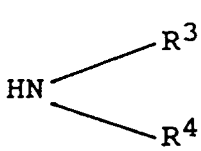

(III),

and any ester groups that may still be present are hydrolysed,
the acids of the general formula I so obtained wherein X represents a hydrogen atom are, if desired, reacted in a manner known per se with at least one metal oxide or metal salt of an element of atomic

numbers 21-29, 31, 32, 38, 39, 42-44, 49, 57-83, and subsequently, if desired, acidic hydrogen atoms present are replaced by cations of inorganic and/or organic bases or amino acids.

**11.** Process for the production of diagnostic media according to claim 7, characterised in that the complex compound, dissolved or suspended in water or physiological saline solution, is brought into a form suitable for enteral or parenteral administration, optionally together with the additives customary in galenic pharmacy.

**12.** $N^3$-(2,6-Dioxomorpholinoethyl-$N^6$-(ethoxycarbonylmethyl)-3,6-diazaoctanedioic acid.

**13.** N-Methylglucamine salt of the gadolinium(III) complex of $N^3$,$N^6$-bis(carboxymethyl)-$N^9$-diethylcarbamoylmethyl-3,6,9-triazaundecanedioic acid,
gadolinium(III) complex of $N^6$-carboxymethyl-$N^3$,$N^9$-bis(dimethylcarbamoylmethyl)-3,6,9-triazaundecanedioic acid,
sodium salt of the dysprosium(III) complex of $N^3$,$N^6$-bis(carboxymethyl)-$N^9$-phenylcarbamoylmethyl-3,6,9-triazaundecanedioic acid,
gadolinium(III) complex of $N^6$-carboxymethyl-$N^3$,$N^9$-bis(N-methyl-N-n-propylcarbamoylmethyl)-3,6,9-triazaundecanedioic acid,
gadolinium(III) complex of $N^6$-carboxymethyl-$N^3$,$N^9$-bis(diethylcarbamoylmethyl)-3,6,9-triazaundecanedioic acid,
gadolinium(III) complex of $N^6$-carboxymethyl-$N^3$,$N^9$-bis(dimethylcarbamoylmethyl)-3,6,9-triazaundecanedioic acid,
manganese(II) complex of trans-1,2-diamino-N,N'-bis-(carboxymethyl)-N,N'-bis-(dimethylcarbamoylmethyl)cyclohexane.

**14.** $N^6$-Carboxymethyl-$N^3$-ethoxycarbonylmethyl-$N^9$-3-oxapentamethylenecarbamoylmethyl-3,6,9-triazaundecanedioic acid,
$N^3$,$N^6$-bis(carboxymethyl)-$N^9$-3-oxapentamethylenecarbamoyl-3,6,9-triazaundecanedioic acid,
sodium salt of the gadolinium(III) complex of $N^3$,$N^6$-bis(carboxymethyl)-$N^9$-3-oxapentamethylenecarbamoylmethyl-3,6,9-triazaundecanedioic acid,
gadolinium(III) complex of $N^6$-carboxymethyl-$N^3$,$N^9$-bis(3-oxapentamethylenecarbamoylmethyl)-3,6,9-triazaundecanedioic acid,
gadolinium(III) complex of $N^6$-carboxymethyl-$N^3$,$N^9$-bis(tetramethylenecarbamoylmethyl)-3,6,9-triazaundecanedioic acid,
gadolinium(III) complex of $N^6$-carboxymethyl-$N^3$,$N^9$-bis(pentamethylenecarbamoylmethyl)-3,6,9-triazaundecanedioic acid,
gadolinium(III) complex of $N^6$-carboxymethyl-$N^3$,$N^9$-bis[N,N-3-(2-hydroxyethyl)-3-azapentamethylenecarbamoylmethyl]-3,6,9-triazaundecanedioic acid,
gadolinium(III) complex of $N^6$-carboxymethyl-$N^3$,$N^9$-bis[N,N-(1-hydroxymethyl)-pentamethylenecarbamoylmethyl]-3,6,9-triazaundecanedioic acid,
sodium salt of the gadolinium(III) complex of $N^3$,$N^6$-bis(carboxymethyl)-$N^9$-(3-oxapentamethylene)-carbamoylmethyl-3,6,9-triazaundecanedioic acid,
manganese(II) complex of trans-1,2-diamino-N,N'-bis-(carboxymethyl)-N,N'-bis(3-oxapentamethylenecarbamoylmethyl)cyclohexane,
gadolinium(III) complex of 3,6,9-tris(carboxymethyl)-3,6,9-triazaundecanedioic acid N,N'-bis(2-carbamoylpentamethylene)diamide,
gadolinium(III) complex of 3,6,9-tris(carboxymethyl)-3,6,9-triazaundecanedioic acid N,N'-bis(3-carbamoylpentamethylene)diamide.

## Revendications

**1.** Composés physiologiquement tolérables répondant à la formule générale I

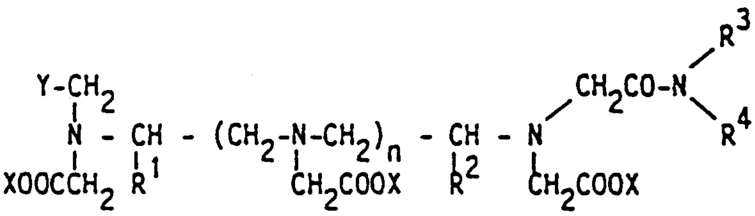

(I),

dans laquelle n est égal à 0,1 ou 2,

$R^1$ et $R^2$ désignent des atomes d'hydrogène, des groupes alkyle inférieur, des groupes phényle, des groupes benzyle, ou bien, lorsque n désigne le chiffre 0, ils désignent en commun un groupe triméthylène ou tétraméthylène,

$R^3$ désigne un radical hydrocarboné aliphatique saturé, insaturé, linéaire ou ramifié ou cyclique ayant jusqu'à 16 atomes de carbone ou, lorsque $R^4$ est un atome d'hydrogène, un groupe cycloalkyle ou un groupe aryle ou aralkyle substitué le cas échéant par un ou plusieurs groupes (dialkylamino en $C_1$ $C_6$) ou par un ou plusieurs groupes alcoxyle en $C_1$ $C_6$,

$R^4$ est un atome d'hydrogène, un radical hydrocarboné saturé, insaturé, linéaire ou ramifié ou cyclique ayant jusqu'à 16 atomes de carbone, ou

$R^3$ et $R^4$ forment ensemble un cycle à 5 ou 6 maillons, saturé ou insaturé, qui renferme éventuellement un atome d'azote supplémentaire, un atome d'oxygène, un atome de soufre ou un radical carbonyle et qui porte éventuellement un ou plusieurs radicaux alkyles en $C_1$-$C_6$ ou hydroxy-alkyles en $C_1$-$C_5$, un radical acyle en $C_2$-$C_6$ éventuellement porteur d'un hydroxy ou d'un alcoxy en $C_1$-$C_6$, un hydroxy,un carbamoyle, un alkyle en $C_1$-$C_6$ porteur d'un carbamoyle, un carbamoyle dont l'azote porte un ou deux alkyles en $C_1$-$C_6$ (lesquels peuvent également former un cycle contenant éventuellement un atome d'oxygène), ou un radical acylamino en $C_1$-$C_6$ ou alkylamino en $C_1$-$C_6$,

X désigne un atome d'hydrogène et/ou un équivalent d'ion métallique, et

Y désigne un groupe

COOX- ou 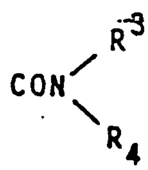

sous réserve que lorsque $R^1$ et $R^2$ représentent des atomes d'hydrogène, $R^3$ et $R^4$ ne représentent pas simultanément des radicaux hydrocarbonés aliphatiques saturés, insaturés, linéaires ou ramifiés ayant jusqu'à 16 atomes de carbone,

ainsi que leurs sels avec des bases organiques et/ou minérales.

**2.** Composés biologiquement tolérables conformes à la revendication 1, caractérisés en ce que X représente des atomes d'hydrogène.

**3.** Composés physiologiquement tolérables selon la revendication 1, caractérisés en ce qu'au moins 2 des substituants X sont des équivalents d'ions métalliques d'au moins 1 élément ayant comme numéro atomique 21-29,42,44 ou 57-83.

**4.** Composés physiologiquement tolérables selon la revendication 1, caractérisés en ce qu'au moins 2 des substituants X sont des équivalents d'ions métalliques d'un radionucléide d'au moins un élément de numéro atomique 27, 29, 31, 32, 38, 39, 43, 49, 62, 64, 70 ou 77.

**5.** Composés physiologiquement tolérables selon la revendication 1, caractérisés en ce que Y représente un groupe COOX.

**6.** Composés physiologiquement tolérables selon la revendication 1, caractérisés en ce que Y désigne un groupe

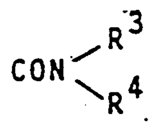

7. Agents de diagnostic contenant au moins un composé physiologiquement tolérable selon les revendications 1 à 6, le cas échéant avec les additifs habituels en pharmacie galénique.

8. Utilisation d'au moins un composé physiologiquement tolérable selon les revendications 3,5 et 6 pour la préparation d'agents pour le diagnostic par RMN ou rayons X.

9. Utilisation d'au moins un composé physiologiquement tolérable selon les revendications 4 à 6 pour la préparation d'agents pour le radiodiagnostic et la radiothérapie.

10. Procédé pour la préparation de composés complexes physiologiquement tolérables répondant à la formule générale I selon la revendication 1, caractérisé en ce que, d'une manière connue en soi,
a) pour la préparation de composés répondant à la formule I dans lesquels Y désigne un groupe,

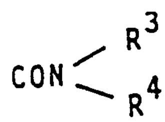

on fait réagir un composé répondant à la formule générale II

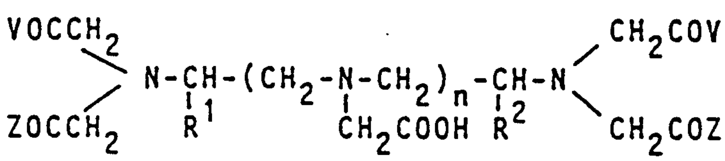 (II)

dans laquelle $R^1$, $R^2$ et n ont les significations indiquées ci-dessus et V et Z représentent en commun un atome d'oxygène ou V un groupe hydroxy et Z le groupe $OR^5$, avec une amine répondant à la formule générale III

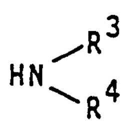 (III)

dans laquelle $R^3$ et $R^4$ ont la signification indiquée ci-dessus, ou
b) pour la préparation de composés répondant à la formule générale I, dans lesquels Y désigne un groupe COOH, on fait réagir un composé répondant à la formule générale VI

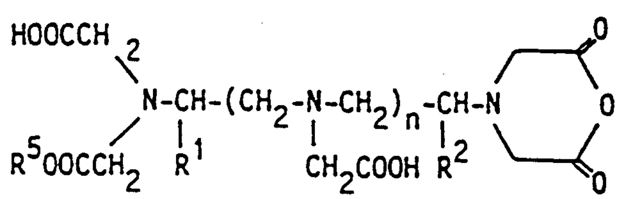 (VI)

ou V

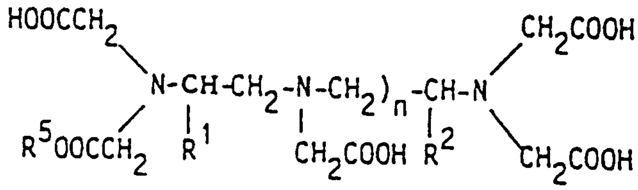

(V)

dans laquelle $R^5$ représente un radical alkyle en $C_1$-$C_6$ et $R^1$, $R^2$ et n ont les significations indiquées dans la revendication 1, avec une amine répondant à la formule générale III

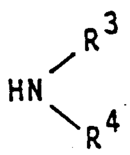

(III)

et on saponifie les groupes ester qui sont éventuellement encore présents,

on fait réagir si on le désire les acides ainsi obtenus répondant à la formule générale I dans laquelle X désigne un atome d'hydrogène, d'une manière connue en soi, avec au moins un oxyde métallique ou un sel métallique d'un élément ayant les numéros atomiques 21-29, 31, 32, 38, 39, 42-44, 49, 57-83 puis, si on le désire, on substitue les atomes d'hydrogène acides présents par des cations de bases minérales et/ou organiques ou d' aminoacides.

**11.** Procédé de préparation des agents de diagnostic selon la revendication 7, caractérisé en ce qu'on amène sous une forme appropriée pour l'administration entérale ou parentérale le composé complexe dissous ou mis en suspension dans de l'eau ou une solution saline physiologique, le cas échéant avec les additifs habituels en pharmacie galénique.

**12.** Acide $N^3$-(2,6-dioxomorpholinoéthyl-$N^6$-(éthoxycarbonylméthyl)-3,6-diazaoctanedioïque.

**13.** Sel de N-méthylglucamine du complexe de gadolinium (III) de l'acide $N^3$, $N^6$-bis-(carboxyméthyl)-$N^9$-diéthylcarbamoylméthyl-3,6,9-triazaundécanedioïque.

Complexe de gadolinium-(III) de l'acide $N^6$-carboxyméthyl-$N^3$, $N^9$-bis(diméthylcarbamoylméthyl)-3,6,9-triazaundécanedioïque.

Sel de sodium du complexe de dysprosium (III) de l'acide $N^3$, $N^6$-bis-(carboxyméthyl)-$N^9$-phénylcarbamoylméthyl-3,6,9-triazaundécanedioïque.

Complexe de gadolinium-(III) de l'acide $N^6$-carboxyméthyl)-$N^3$, $N^9$-bis(N-méthyl-N-n-propylcarbamoylméthyl-3,6,9-triazaundécanedioïque.

Complexe de gadolinium-(III) de l'acide $N^6$-carboxyméthyl-$N^3$, $N^9$-bis(diéthyl-carbamoylméthyl)-3,6,9-triazaundécanedioïque.

Complexe de gadolinium-(III) de l'acide $N^6$-carboxyméthyl-$N^3$, $N^9$-bis(diméthyl-carbamoylméthyl)-3,6,9-triazaundécanedioïque.

Complexe de manganèse (II) du trans-1,2-diamino-N,N'-bis(carboxyméthyl)-N,N'-bis-(diméthylcarbomoylméthyl)-cyclohexane.

**14.** Acide $N^6$ carboxyméthyl-$N^3$-éthoxycarbonylméthyl-$N^9$-3-oxapentaméthylène-carbamoylméthyl-3,6,9-triazaundécanedioïque.

Acide $N^3$,$N^6$ bis (carboxyméthyl)-$N^9$-3-oxapentaméthylène-carbamoyl-3,6,9-triazaundécanedioïque.

Sel de sodium du complexe de gadolinium-(III) de l'acide $N^3$, $N^6$-bis(carboxyméthyl)-$N^9$-3-oxapentaméthylène-carbamoylméthyl-3,6,9-triazaundécanedioïque.

Complexe de gadolinium-(III) de l'acide $N^6$-carboxyméthyl-$N^3$,$N^9$-bis-(3-oxapentaméthylène-carbamoylméthyl)-3,6,9-triazaundécanedioïque.

Complexe de gadolinium-(III) de l'acide $N^6$-carboxyméthyl-$N^3$,$N^9$-bis-(tétraméthylène-carbamoylméthyl)-3,6,9-triazaundécanedioïque.

Complexe de gadolinium-(III) de l'acide $N^6$-carboxyméthyl-$N^3$,$N^9$-bis-(pentaméthylène-carbamoylméthyl)-3,6,9-triazaundécanedioïque

Complexe de gadolinium-(III) de l'acide $N^6$-carboxyméthyl-$N^3$,$N^9$-bis-[N,N-3-(2-hydroxyéthyl)-3-azapentaméthylène-carbamoylméthyl]-3,6,9-triazaundécanedioïque.

Complexe de gadolinium-(III) de l'acide $N^6$-carboxyméthyl-$N^3$,$N^9$-bis-[N,N-(1-hydroxyméthyl)-pentaméthylène-carbamoylméthyl]-3,6,9-triazaundécanedioïque.

Sel de sodium du complexe de gadolinium-(III) de l'acide $N^3$,$N^6$-bis-(carboxyméthyl)-$N^9$-(3-oxapentaméthylène)-carbamoylméthyl-3,6,9-triazaundécanedioïque.

Complexe de Mn (II) du trans-1,2-diamino-N,N'-bis-(carboxyméthyl)-N,N'-bis-(3-oxapentaméthylène-carbamoylméthyl)-cyclohexane

Complexe de gadolinium-(III) du N,N'-bis-(2-carbamoylpentaméthylène)-diamide de l'acide 3,6,9-tris-(carboxyméthyl)-3,6,9-triazaundécanedioïque

Complexe de gadolinium)-(III) du N,N'-bis-(3-carbamoylpentaméthylène)-diamide de l'acide 3,6,9-tris-(carboxyméthyl)-3,6,9-triazaundécanedioïque.